# DEMANDE DE BREVET EUROPEEN

(11) **EP 1 634 580 A1**
(43) Date de publication de la demande: **15.03.2006**
(21) Numéro de dépôt: 05300730.8
(22) Date de dépôt: 08.09.2005
(51) Int. Cl.: A61K 8/81, A61Q 5/06

(54) **COMPOSITION DE COIFFAGE COMPRENANT L'ASSOCIATION D'UN MATERIAU POLYMERIQUE HYDROPHILE DE HAUTE POROSITE ET D'UN POLYMERE FIXANT**

(30) Priorité: 09.09.2004 FR 0452007
(71) Demandeur: L'OREAL, 75008 Paris (FR)
(72) Inventeur: Mathonneau, Estelle, 75010, PARIS (FR); Rollat, Isabelle, 75017, PARIS (FR)
(74) Mandataire: Catherine, Alain

(57) **Abrégé**

L'invention concerne une composition de coiffage comprenant dans un milieu liquide physiologiquement acceptable, l'association :
- d'au moins un matériau polymérique hydrophile de haute porosité, à l'exclusion des polymères siliconés, et des polymères polyméthacrylate d'alkyle (PMMA), et
- d'au moins un polymère fixant.

La composition de l'invention permet d'obtenir un coiffage avec une meilleure fixation et durable dans le temps.

## Description

La présente invention concerne une composition de coiffage des cheveux comprenant l'association d'un matériau de haute porosité et d'un polymère fixant.

Il existe de nombreux produits de coiffage sous forme de laques, de sprays, de gels ou de lotions. Ces produits sont des solutions ou dispersions de polymères qui, après évaporation de la phase solvant, assurent la fixation et le maintien de la coiffure grâce à la formation de films polymériques entourant chaque cheveu à la manière d'une gaine et grâce à l'établissement de liens physiques entre les cheveux.

En particulier, les produits de coiffage permettent de modeler et fixer la coiffure grâce à des polymères fixants, collants, tackants, à base de corps gras, etc. Ces produits doivent permettre de fixer la coiffure de façon satisfaisante et durablement dans le temps.

L'un des problèmes lié à ces produits de coiffage est l'efficacité dans le temps.

Pour améliorer l'efficacité dans le temps, on connaît par ailleurs des traitements permanents des fibres kératiniques. Ces traitements utilisent un agent réducteur et un agent oxydant, et nécessitent une mise sous tension mécanique des cheveux à l'aide d'un matériel d'enroulage afin de conférer une mise en forme durable de la chevelure.

Bien qu'ils permettent effectivement de fixer la coiffure de manière plus durable que les gels associés à des polymères fixants, ces procédés présentent l'inconvénient de dégrader le toucher de la fibre et sont des procédés relativement longs.

La demanderesse a découvert de façon fortuite que l'utilisation de matériaux polymériques de haute porosité en association avec des polymères filmogènes permet d'obtenir un coiffage avec une meilleure fixation et durable dans le temps.

La présente invention a par conséquent pour objet une composition de coiffage comprenant dans un milieu liquide physiologiquement acceptable, l'association :
- d'au moins un matériau polymérique hydrophile de haute porosité, à l'exclusion des polymères siliconés, et des polymères polyméthacrylate d'alkyle (PMMA), et
- d'au moins un polymère fixant.

Par polymère de type polyméthacrylate d'alkyle (PMMA), on entend les homopolymères issus uniquement de réactions de polymérisation de fonctions méthacryliques.

Par matériau polymérique hydrophile, on entend, au sens de la présente invention, un matériau comprenant un polymère dont au moins un monomère est hydrosoluble.

Un monomère « hydrosoluble » au sens de la présente invention, est un monomère qui, lorsqu'on l'introduit dans de l'eau à une température de 25°C et à une concentration en poids égale à 0,5%, éventuellement neutralisé, permet l'obtention d'une solution macroscopiquement homogène et transparente, c'est-à-dire ayant une valeur de transmittance de la lumière, à une longueur d'onde égale à 500 nm, à travers un échantillon de 1 cm d'épaisseur, d'au moins 70%, de préférence d'au moins 80%.

De préférence, le matériau polymérique de haute porosité est solide, sous forme de particules. Les particules peuvent être de toutes formes et en particulier sphériques, elliptiques ou polyhexagonales.

La section des particules de matériau polymérique hydrophile est de préférence comprise entre 0,5 nm et 250 µm et de préférence comprise entre 10 nm à 80 µm.

Par matériau de haute porosité, on entend un matériau présentant une surface spécifique supérieure à 0,1 m²/g.

Le matériau polymérique hydrophile possède de préférence une porosité telle que les surfaces spécifiques sont comprises entre 0,1 et 1000 m²/g .

Les particules de haute porosité sont capables d'absorber le polymère auquel elles sont associées, et de créer un revêtement sur le cheveu ayant des « éponges de polymère » c'est-à-dire des particules absorbantes imbibées de polymère qui permettent d'augmenter significativement la quantité de polymère déposée, la qualité du dépôt et donc, pour une application de coiffage, d'améliorer la fixation de la coiffure.

Ce type de polymère de haute porosité permet également d'apporter une texture particulière à la composition cosmétique.

Les monomères hydrophiles utilisés dans la présente invention peuvent être de nature anionique, non-ionique ou cationique et peuvent être utilisés seuls ou sous forme de mélange contenant deux ou plusieurs monomères différents.

On peut citer à titre d'exemples de monomères hydrophiles anioniques, les acides carboxyliques à insaturation éthylénique, tels que l'acide acrylique, l'acide méthacrylique, l'acide itaconique, l'acide fumarique, l'acide crotonique et l'acide maléique, l'acide 2-acrylamido-2-méthylpropane-sulfonique, l'acide styrènesulfonique, l'acide vinylsulfonique et l'acide vinylphosphonique et leurs sels.

Les monomères hydrophiles non-ioniques englobent, entre autres, l'acrylamide, les acrylamides N-alkylés en C₁₋₆ ou N,N-dialkylés en C₁₋₃, l'acrylate de polyéthylèneglycol, le méthacrylate de polyéthylèneglycol, le N-vinylacétamide, le N-méthyl-N-vinylacétamide, le N-vinylformamide, le N-méthyl-N-vinylformamide, les N-vinyllactames comportant un groupe cyclique de 4 à 9 atomes de carbone, l'alcool vinylique (copolymérisé sous forme d'acétate de vinyle puis hydrolysé), l'oxyde d'éthylène, l'acrylate d'hydroxyéthyle, l'acrylate d'hydroxypropyle, le méthacrylate d'hydroxyéthyle, le méthacrylate d'hydroxypropyle, le méthacrylate de méthyle, et la N-acryloyl morpholine.

Enfin, les monomères hydrophiles cationiques englobent par exemple le chlorure de diméthyldiallylammonium, le chlorure de méthylvinylimidazolium, la 2-vinylpyridine, la 4-vinylpyridique, la 2-méthyl-5-vinylpyridine, les halogénures de N-(alkyle en C₁₋₄)-4-vinylpyridinium tels que l'iodure de N-méthyl-4-vinylpyridinium, la vinylamine, les monomères de formule :

**H**_{**2**}**C=CR**_{**1**}**-CO-X**_{**2**}

dans laquelle:
R₁ représente un atome d'hydrogène ou un groupe méthyle,
X₂ représente un groupe hydrocarboné linéaire ou ramifié en C₁₋₆ portant au moins une fonction amine primaire, secondaire ou tertiaire ou au moins un atome d'azote quaternaire, ou un groupe de formule NHR₂ ou de formule NR₂R₃ où R₂ et R₃ représentent indépendamment l'un de l'autre chacun un groupe hydrocarboné en C₁₋₆, linéaire ou ramifié, portant au moins une fonction amine primaire, secondaire ou tertiaire ou au moins un atome d'azote quaternaire.

Le matériau polymérique peut contenir de 0,1 à 40% d'un ou plusieurs monomères hydrophobes (solubilité inférieure à 0,5 dans l'eau à 25°C) qui sont choisis de préférence parmi les monomères vinylaromatiques tels que le styrène et ses dérivés alkylés comme le 4-butylstyrène, l'α-méthylstyrène et le vinyltoluène, les diènes tels que le butadiène et le 1,3-hexadiène, et les dérivés alkylés des diènes tels que l'isoprène et le diméthylbutadiène, le chloroprène, les acrylates d'alkyle en C₂₋₁₀, d'aryle en C₆₋₁₀ ou d'aralkyle en C₇₋₂₀ et les méthacrylates d'alkyle en C₂₋₁₀, d'aryle en C₆₋₁₀ ou d'aralkyle en C₇₋₂₀, comme par exemple les (meth)acryates de méthyle, d'éthyle, de n-butyle, de 2-éthylhexyle, de tert-butyle, d'isobornyle, de phényle ou de benzyle, l'acétate de vinyle, les vinyléthers de formule CH₂=CH-O-R et les allyléthers de formule CH₂=CH-CH₂-O-R où R représente un groupe alkyle en C₁₋₆, l'acrylonitrile, le chlorure de vinyle, le chlorure de vinylidène, la caprolactone, l'éthylène, le propylène, les monomères vinyliques fluorés ou à chaîne perfluorée, tels que les acrylates et méthacrylates de fluoroalkyle ou les α-fluoroacrylates d'alkyle.

Le matériau polymérique hydrophile de haute porosité peut être un homopolymère ou un copolymère, y compris un copolymère bloc ou non.

Des procédés de réalisation d'un matériau polymérique de haute porosité sont décrits notamment dans les demandes US 6,048 908, et US 6,218,440.

De tels produits sont aussi décrits dans la demande WO 2003/ 091321 et la publication « Preparation and characterisation of thermosensitive beads with macroporous structures", Caihua Ni - Zheng WANG 6 Xiao Xia Zhu, J. of Applied Polymer Science, vol. 91, 1792-1797 (2004)

Par polymère fixant, on entend au sens de la présente invention tout polymère susceptible d'apporter et/ou de maintenir une forme à une chevelure.

On peut utiliser pour la présente invention tout polymère fixant filmogène connu en tant que tel dans le domaine des traitements capillaires, ainsi que bien entendu également des mélanges contenant plusieurs de ces polymères. On distingue classiquement les polymères fixants cationiques, anioniques, amphotères et non ioniques.

Les polymères fixants cationiques utilisables selon la présente invention sont de préférence choisis parmi les polymères comportant des groupements amine primaire, secondaire, tertiaire et/ou quaternaire faisant partie de la chaîne polymère ou directement reliés à celle-ci, et ayant un poids moléculaire compris entre 500 et environ 5 000 000 et de préférence entre 1000 et 3 000 000.

Parmi ces polymères, on peut citer plus particulièrement les polymères cationiques suivants :
(1) les homopolymères ou copolymères dérivés d'esters ou d'amides acryliques ou méthacryliques et comportant au moins un des motifs suivants : 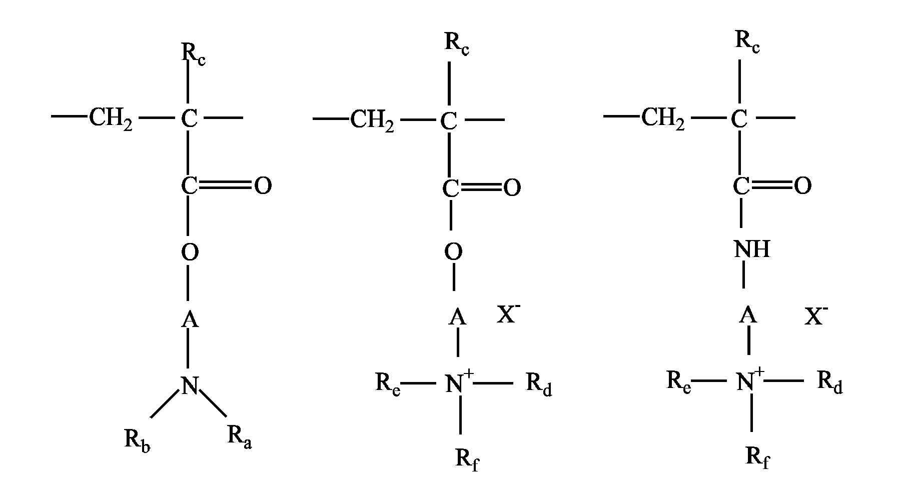
   dans lesquels :
   Rₐ et R_{b} représentent chacun un atome d'hydrogène ou un groupe alkyle en C₁₋₆,
   R_{c} désigne un atome d'hydrogène ou un radical CH₃ .
   R_{d}, Rₑ et R_{f}, identiques ou différents, représentent chacun un groupe alkyle en C₁₋₁₈ ou un radical benzyle,

   A est un groupe alkyle linéaire ou ramifié en C₁₋₆ ou un groupe hydroxyalkyle en C₁₋₄, et
   X- désigne un anion méthosulfate ou halogénure tel qu'un ion chlorure ou bromure.
   Les copolymères de la famille (1) contiennent en outre un ou plusieurs motifs dérivant de comonomères pouvant être choisis dans la famille des acrylamides, méthacrylamides, diacétone-acrylamides, acrylamides et méthacrylamides substitués sur l'azote par des groupes alkyle inférieurs, des acides acryliques ou méthacryliques ou leurs esters, des vinyllactames tels que la vinylpyrrolidone ou le vinylcaprolactame, et des esters vinyliques.
   Ainsi, on peut citer parmi les copolymères de la famille (1) :
   - les copolymères d'acrylamide et de méthacrylate de diméthylaminoéthyle quaternisés au sulfate de diméthyle ou avec un halogénure de diméthyle, tels que celui vendu sous la dénomination HERCOFLOC® par la Société HERCULES,
   - les copolymères d'acrylamide et de chlorure de méthacryloyloxyéthyltriméthylammonium décrits par exemple dans la demande de brevet EP-A-080976 et vendus sous la dénomination BINA QUAT® P 100 par la Société CIBA GEIGY,
   - le copolymère d'acrylamide et de méthosulfate de méthacryloyloxyéthyltriméthylammonium vendu sous la dénomination RETEN® par la Société HERCULES,
   - les copolymères vinylpyrrolidone/acrylate ou méthacrylate de dialkylaminoalkyle quaternisés ou non, tels que les produits vendus sous la dénomination GAFQUAT® par la Société ISP, comme par exemple GAF-QUAT® 734 OU GAFQUAT® 755, ou bien les produits dénommés COPOLYMER® 845, 958 et 937. Ces polymères sont décrits en détail dans les demandes de brevets français FR2.077.143 et FR2.393.573,
   - les terpolymères méthacrylate de diméthyl-aminoéthyle/vinylcaprolactame/vinylpyrrolidone tels que le produit commercialisé sous la dénomination GAFFIX VC 713 par la Société ISP, et
   - le copolymère vinylpyrrolidone/méthacrylamide de diméthylaminopropyle quaternisé tel que le produit vendu sous la dénomination GAFQUAT® HS 100 par la Société ISP.
(2) les polysaccharides quaternisés décrits plus particulièrement par les brevets américains 3.589.578 et 4.031.307 tels que les gommes de guar contenant des groupements cationiques trialkylammonium.
   De tels produits sont commercialisés notamment sous les dénominations commerciales de JAGUAR® C13 S, JAGUAR® C15 et JAGUAR® C17 par la Société MEYHALL.
(3) Les copolymères quaternaires de vinylpyrrolidone et de vinylimidazole tels que les produits commercialisés par BASF sous la dénomination LUVIQUAT® TFC,
(4) les chitosanes ou leurs sels, en particulier les acétate, lactate, glutamate, gluconate ou pyrrolidonecarboxylate de chitosane.
   On peut citer le chitosane ayant un taux de désacétylation de 90,5% en poids vendu sous la dénomination KYTAN BRUT STANDARD® par la Société ABER TECHNOLOGIES, le pyrrolidonecarboxylate de chitosane vendu sous la dénomination KYTAMER® PC par la Société AMERCHOL.
(5) Les dérivés de cellulose cationiques tels que les copolymères de cellulose ou les dérivés de cellulose greffés avec un monomère hydrosoluble comportant un groupe ammonium quaternaire et décrits notamment dans le brevet US 4 131 576 tels que les hydroxyalkylcelluloses, comme les hydroxyméthylhydroxyéthyl- ou hydroxypropylcelluloses greffées notamment avec un sel de méthacryloyloxyéthyltriméthylammonium, de méthacrylamidopropyltriméthylammonium ou de diméthyldiallylammonium.

Les produits commercialisés répondant à cette définition sont plus particulièrement les produits vendus sous les dénominations CELQUAT® L 200 et CELQUAT® H 100 par la Société NATIONAL STARCH.

Les polymères fixants anioniques généralement utilisés sont des polymères comportant des groupements dérivés d'un acide carboxylique, sulfonique ou phosphorique et ont un poids moléculaire moyen en poids compris entre environ 500 et 5 000 000.

Les groupements acide carboxyliques sont apportés par des monomères insaturés contenant une ou deux fonctions acide carboxylique, tels que ceux répondant à la formule : 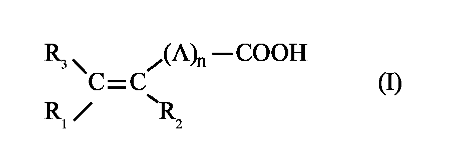
dans laquelle n est un nombre entier de 0 à 10, A désigne un groupement méthylène, éventuellement relié à l'atome de carbone du groupement insaturé ou du groupement méthylène voisin, lorsque n est supérieur à 1, par l'intermédiaire d'un hétéroatome tel que oxygène ou soufre, R₃ désigne un atome d'hydrogène, un groupement phényle ou benzyle, R₁ désigne un atome d'hydrogène, un groupement alkyle inférieur ou carboxyle, et R₂ désigne un atome d'hydrogène, un groupement alkyle inférieur, un groupement ―CH₂-COOH, phényle ou benzyle.

Dans la formule précitée un radical alkyle inférieur désigne de préférence un groupement ayant de 1 à 4 atomes de carbone et en particulier un groupe méthyle ou éthyle.

Les polymères fixants anioniques carboxylés préférés selon l'invention sont :
A) les homo- ou copolymères d'acide acrylique ou méthacrylique ou leurs sels et en particulier les produits commercialisés sous les dénominations VERSICOL® E ou K par la Société ALLIED COLLOID, et sous la dénomination ULTRAHOLD® par la Société BASF ; les copolymères d'acide acrylique et d'acrylamide commercialisés sous forme de sel de sodium sous les dénominations RETEN® 421, 423 ou 425 par la Société HERCULES ; les sels de sodium des acides polyhydroxycarboxyliques.
B) Les copolymères d'acide acrylique ou d'acide méthacrylique et d'un monomère monoéthylénique tel que l'éthylène, le styrène, les esters vinyliques, les esters d'acide acrylique ou méthacrylique.
   Ces copolymères peuvent être greffés sur un polyalkylène glycol tel que le polyéthylèneglycol et sont éventuellement réticulés.
   De tels polymères sont décrits en particulier dans la demande de brevet français FR 1 222 944 et dans la demande de brevet allemand DE 2 330 956. On peut notamment citer les copolymères comportant dans leur chaîne un motif acrylamide éventuellement N-alkylé et/ou hydroxyalkylé, tels que ceux décrits dans les demandes de brevet luxembourgeois LU 75370 et LU75371 ou proposés sous la dénomination QUADRAMER® par la Société AMERICAN CYANAMID.
   On peut également citer les copolymères d'acide acrylique et de méthacrylate d'alkyle en C₁-C₄, et les terpolymères de vinylpyrrolidone, d'acide (méth)acrylique et de (méth)acrylate d'alkyle en C₁-C₂₀, par exemple de lauryle (ACRYLDONE® LM de la Société ISP), de tertiobutyle (LUVIFLEX® VBM 70 commercialisé par BASF) ou de méthyle (STEPANHOLD® EXTRA commercialisé par STEPAN), et les terpolymères acide méthacrylique/acrylate d'éthyle/acrylate de tertiobutyle tel que le produit commercialisé sous la dénomination LUVIMER® 100 P par la Société BASF.
C) Les copolymères dérivés d'acide crotonique tels que ceux comportant dans leur chaîne des motifs acétate ou propionate de vinyle et éventuellement d'autres monomères tels que les esters allylique, méthallylique ou vinylique d'un acide carboxylique saturé, linéaire ou ramifié, à longue chaîne hydrocarbonée comportant au moins 5 atomes de carbone, ces polymères pouvant éventuellement être greffés et réticulés, ou encore les esters vinylique, allylique ou méthallylique d'un acide carboxylique α- ou β-cyclique.
   De tels polymères sont décrits, entre autres, dans les demandes de brevets français FR 1 222 944, FR 1 580 545, FR2 265 782, FR 2 265 781, FR 1 564 110 et FR 2 439 798.
   On peut citer à titre d'exemples de produits commerciaux entrant dans cette classe les résines 28-29-30, 26-13-14 et 28-13-10 commercialisées par la Société NATIONAL STARCH.
D) Les copolymères dérivés d'acides ou d'anhydrides carboxyliques monoinsaturés en C₄-C₈ choisis parmi :
   - les copolymères comprenant :
      (ii) un ou plusieurs acides ou anhydrides maléique, fumarique, itaconique et
      (iii) au moins un monomère choisi parmi les esters vinyliques, les éthers vinyliques, les halogénures vinyliques, les dérivés phénylvinyliques, l'acide acrylique et ses esters, les fonctions anhydride de ces copolymères étant éventuellement monoestérifiées ou monoamidifiées.

   De tels polymères sont décrits en particulier dans les demandes de brevets US 2,047,398, US 2,723,248, US 2,102,113 et GB 839,805 et notamment ceux commercialisés sous les dénominations GANTREZ AN ou ES, AVANTAGE® CP par la Société ISP ;
   - les copolymères comprenant (i) un ou plusieurs anhydrides maléique, citraconique ou itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne, les fonctions anhydrides de ces copolymères étant éventuellement monoestérifiées ou monoamidifiées.

   Ces polymères sont par exemple décrits dans les demandes de brevets français FR 2 350 384 et FR 2 357 241 de la demanderesse.
E) Les polyacrylamides comportant des groupements carboxylate.

Les groupements anioniques des polymères fixants anioniques de la présente invention peuvent également être des groupes acide sulfonique apportés par des motifs vinylsulfonique, styrènesulfonique, naphtalènesulfonique ou acrylamidoalkylsulfonique.

Ces polymères à groupes acide sulfonique sont notamment choisis parmi:
- les sels de poly(acide vinylsulfonique) ayant un poids moléculaire moyen en poids compris entre environ 1000 et 100 000 ainsi que les copolymères d'acide vinylsulfonique et d'un comonomère insaturé tel que l'acide acrylique, l'acide méthacrylique, les esters de ces acides, l'acrylamide, les dérivés d'acrylamide, les éthers vinyliques et la vinylpyrrolidone ;
- les sels de poly(acide styrènesulfonique). On peut citer à titre d'exemple deux sels de sodium ayant un poids moléculaire moyen en poids d'environ 500 000 et d'environ 100 000 commercialisés respectivement sous les dénominations FLEXAN® 500 et FLEXAN® 130 par la Société NATIONAL STARCH. Ces composés sont décrits dans le brevet FR 2 198 719 ;
- les sels de poly(acide acrylamidesulfonique), tels que ceux mentionnés dans le brevet US 4,128,631 et plus particulièrement le poly(acide acrylamidoéthyl-propanesulfonique) commercialisé sous la dénomination COSMEDIA POLYMER® HSP 1180 par la Société HENKEL.

Selon l'invention, les polymères fixants anioniques sont de préférence choisis parmi les copolymères d'acide acrylique tels que les terpolymères acide acrylique/acrylate d'éthyle/N-tertiobutylacrylamide commercialisés notamment sous la dénomination ULTRAHOLD STRONG® par la Société BASF, les copolymères dérivés d'acide crotonique tels que les terpolymères acétate de vinyle/tertio-butyl benzoate de vinyle/acide crotonique et les terpolymères acide crotonique/acétate de vinyle/néododécanoate de vinyle commercialisés notamment sous la dénomination Résine 28-29-30 par la Société NATIONAL STARCH, les copolymères dérivés d'acide ou d'anhydride maléique, fumarique ou itaconique et contenant comme comonomères des esters vinyliques, des éthers vinyliques, des halogénures vinyliques, des dérivés phénylvinyliques, l'acide acrylique et les esters d'acide acrylique, tels que les copolymères méthylvinyléther/anhydride maléique monoestérifié, commercialisés par exemple sous la dénomination GANTREZ® par la Société ISP, les copolymères d'acide méthacrylate de méthyle commercialisés sous la dénomination EUDRAGIT®L par la Société ROHM PHARMA, les copolymères acide méthacrylique/méthacrylate de méthyle/acrylate d'alkyle en C₁₋₄/acide acrylique ou méthacrylate d'hydroxyalkyle en C₁₋₄, commercialisés sous la dénomination AMERHOLD® DR 25 par la Société AMERCHOL, ou sous la dénomination ACUDYNE® 255 par la Société ROHM & HAAS, les copolymères d'acide méthacrylique et d'acrylate d'éthyle commercialisés sous la dénomination LUVIMER® MAEX ou MAE par la Société BASF et les copolymères acétate de vinyle/acide crotonique et les copolymères acétate de vinyle/acide crotonique greffés par du polyéthylèneglycol commercialisés sous la dénomination ARISTOFLEX® A par la Société BASF.

Les polymères fixants amphotères utilisables pour la présente invention sont choisis notamment parmi les polymères comportant des motifs B et C répartis statistiquement dans la chaîne polymère, où B désigne un motif dérivant d'un monomère comportant au moins un atome d'azote basique et C désigne un motif dérivant d'un monomère comportant un ou plusieurs groupements acide carboxylique ou acide sulfonique. Les polymères fixants amphotères peuvent également comporter des motifs zwittérioniques de type carboxybétaïne ou sulfobétaïne. Il peut également s'agir de polymères à chaîne principale cationique comportant des groupements amine primaire, secondaire, tertiaire ou quaternaire, parmi lesquels au moins un, porte, par l'intermédiaire d'un radical hydrocarboné, un groupement acide carboxylique ou acide sulfonique. Les polymères fixants amphotères peuvent encore avoir une chaîne anionique dérivée d'acides dicarboxyliques α,β-insaturés dont l'un des groupements carbonyle a été amené à réagir avec une polyamine comportant un ou plusieurs groupements amine primaire ou secondaire.

Les polymères fixants amphotères répondant à la définition donnée ci-dessus sont choisis en particulier parmi les polymères suivants :
(1) Les polymères résultant de la copolymérisation d'un monomère vinylique portant un groupement acide carboxylique tel que l'acide acrylique, l'acide méthacrylique, l'acide maléique, l'acide α-chloroacrylique, et d'un monomère vinylique contenant au moins une fonction basique tel que les méthacrylate et acrylate de dialkylaminoalkyle ou les dialkylaminoalkyl(méth)acrylamides. De tels composés sont décrits par exemple dans la demande de brevet américain US 3,836,537.
(2) Les polymères comportant des motifs dérivés :
   (a) d'au moins un monomère choisi parmi les acrylamides ou méthacrylamides N-alkylés,
   (b) d'au moins un comonomère contenant une ou plusieurs fonctions acide carboxylique, et
   (c) d'au moins un comonomère basique tel que des esters à substituants amine primaire, secondaire, tertiaire ou quaternaire d'acide acrylique et d'acide méthacrylique, et le produit de quaternisation du méthacrylate de diméthylaminoéthyle avec le sulfate de diméthyle ou diéthyle.

   Les acrylamides ou méthacrymlamides N-alkylés (a) préférés sont ceux portant des radicaux alkyle en C₂₋₁₂ , tels que le N-éthylacrylamide, le N-tertiobutylacrylamide, le N-tertio-octylacrylamide, le N-octylacrylamide, le N-décylacrylamide, le N-dodécylacrylamide ainsi que les méthacrylamides correspondants.
   Les comonomères à groupe acide carboxylique (b) sont choisis plus particulièrement parmi les acides acrylique, méthacrylique, crotonique, itaconique, maléique, fumarique ainsi que parmi les monoesters d'alkyle en C₁₋₄ des acides ou des anhydrides maléique ou fumarique.
   Les comonomères basiques (c) préférés sont le méthacrylate d'aminoéthyle, le méthacrylate de butylaminoéthyle, le méthacrylate de N,N'-diméthylaminoéthyle et le méthacrylate de N-tertiobutylaminoéthyle.
   On utilise en particulier les copolymères dont la dénomination CTFA (4^{ième} Ed., 1991) est « Octylacrylamide /acrylates/butylaminoéthylméthacrylate copolymer », tels que les produits commercialisés sous la dénomination AMPHOMER® ou LOVOCRYL® 47 par la Société NATIONAL STARCH.
(3) Les polyaminoamides réticulés et alcoylés, dérivés en partie ou en totalité de polyaminoamides de formule générale :

   **(II) -[C(=0)-R**_{**4**}**-C(=0)-Z-]-**

   dans laquelle R₄ représente un radical divalent dérivé d'un acide dicarboxylique saturé, d'un acide aliphatique mono- ou dicarboxylique à double liaison éthylénique, d'un ester d'alkyle en C₁₋₆ de ces acides ou d'un radical dérivant de l'addition de l'un quelconque desdits acides sur une amine bis primaire ou bis secondaire, et Z désigne un radical d'une polyalkylène-polyamine bis-primaire, mono- ou bis-secondaire, et de préférence représente :
   a) dans les proportions de 60 à 100 % en moles, le radical :

      **(III) -NH-[(CH**_{**2**}**)**_{**X**}**-NH]p-**

      où x = 1 et p = 2 ou 3, ou bien x = 3 et p = 2,
      ce radical dérivant de la diéthylènetriamine, de la triéthylènetétraamine ou de la dipropylènetriamine ;
   b) dans les proportions de 0 à 40% en moles le radical de formule (III) dans lequel x = 2 et p = 1, dérivé de l'éthylènediamine, ou le radical 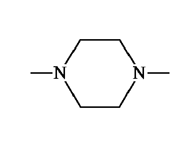 dérivé de la pipérazine :
   c) dans les proportions de 0 à 20 % en moles le radical ―NH-(CH₂)₆₋NH- dérivé de l'hexaméthylènediamine, ces polyaminoamines étant réticulées par addition de 0,025 à 0,35 mole par mole de groupement amine, d'un agent réticulant bifonctionnel choisi parmi les épihalohydrines, les diépoxydes, les dianhydrides, les composés di-insaturés, et alcoylés par l'acide acrylique, l'acide chloracétique ou une alcane-sulfone.

   Les acides carboxyliques saturés sont choisis de préférence parmi les acides ayant 6 à 10 atomes de carbone tels que l'acide adipique, l'acide triméthyl-2,2-4-adipique et triméthyl-2,4,4-adipique, l'acide téréphtalique, les acides à double liaison éthylénique comme par exemple les acides acrylique, méthacrylique, itaconique.
   Les alcane-sulfones utilisées dans l'alcoylation sont de préférence la propanesulfone ou la butanesulfone.
   Les sels des agents d'alcoylation sont de préférence les sels de sodium ou de potassium.
(4) Les polymères comportant des motifs zwittérionique de formule : 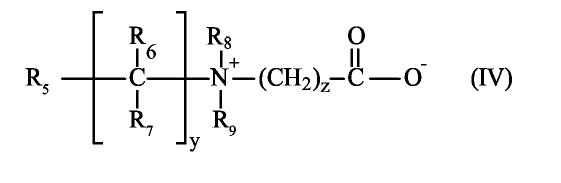 dans laquelle R₅ désigne un groupement insaturé polymérisable tel qu'un groupement acrylate, méthacrylate, acrylamide ou méthacrylamide, y et z représentent chacun un nombre entier de 1 à 3, R₆ et R₇ représentent chacun indépendamment un atome d'hydrogène ou un groupe méthyle, éthyle ou propyle, R₈ et R₉ représentent chacun indépendamment un atome d'hydrogène ou un radical alkyle, le nombre total d'atomes de carbone dans R₈ et R₉ ne dépassant pas 10.
   Les polymères comprenant de tels motifs de formule (IV) peuvent comporter en outre des motifs dérivés de monomères non zwittérioniques tels que l'acrylate ou le méthacrylate de diméthyle ou de diéthylaminoéthyle, les acrylates ou méthacrylates d'alkyle, les acrylamides ou méthacrylamides ou l'acétate de vinyle.
   A titre d'exemple, on peut citer le copolymère méthacrylate de méthyle/méthacrylate de diméthylcarboxyméthylammonioéthyle tel que le produit commercialisé sous la dénomination DIAFORMER® Z301 par la Société SANDOZ.
(5) Les polymères dérivés du chitosane comportant des motifs monomères répondant aux formules suivantes : 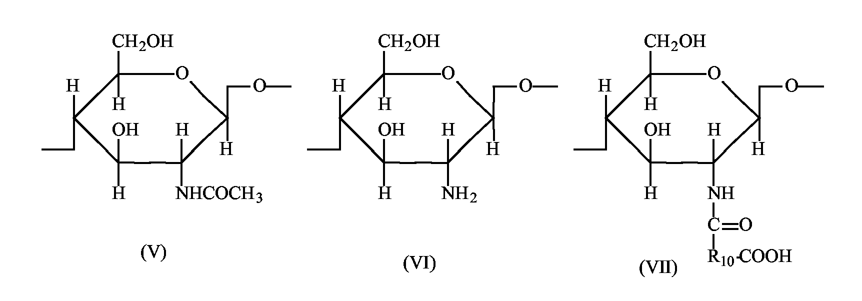 le motif de formule (V) étant présent dans des proportions comprises entre 0 et 30%, le motif de formule (VI) dans des proportions comprises entre 5 et 50% et le motif de formule (VII) dans des proportions comprises entre 30 et 90%, étant entendu que dans ce motif (VII), R₁₀ représente un radical de formule : 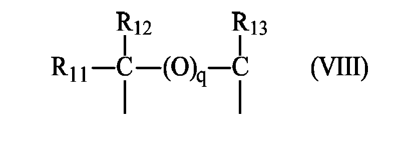 dans laquelle :
   si q = 0, alors R₁₁, R₁₂ et R₁₃, identiques ou différents, représentent chacun un atome d'hydrogène, un groupe méthyle, hydroxyle, acétoxy ou amino, un groupe monoalcoylamine ou dialcoylamine éventuellement interrompu par un ou plusieurs atomes d'azote et/ou éventuellement substitués par un ou plusieurs groupes amine, hydroxyle, carboxyle, alcoylthio ou sulfo, un groupe alcoylthio dont le groupe alcoyle porte un reste amino, l'un au moins des radicaux R₁₁, R₁₂ et R₁₃ étant dans ce cas un atome d'hydrogène ; ou si q = 1, alors R₁₁, R₁₂ et R₁₃ représentent chacun un atome d'hydrogène, ainsi que les sels formés par ces composés avec des bases ou des acides.
(6) Les polymères obtenus par N-carboxyalkylation du chitosane, comme le N-carboxyméthylchitosane ou le N-carboxybutylchitosane commercialisé sous la dénomination EVALSAN® par la Société JAN DEKKER.
(7) Les polymères répondant à la formule générale (IX) : 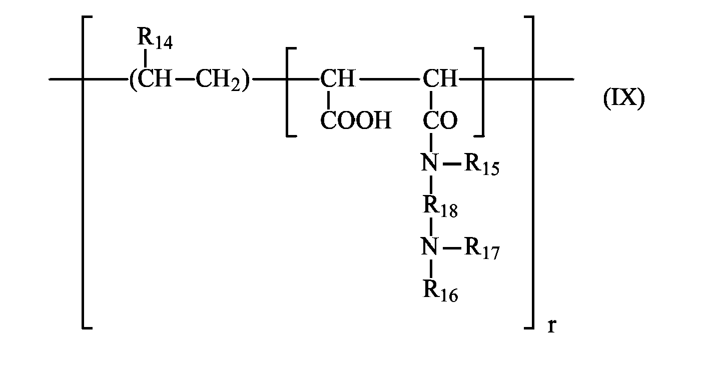 décrits notamment dans la demande de brevet FR 1 400 366, dans laquelle R₁₄ représente un atome d'hydrogène ou un radical CH₃O, CH₃CH₂O ou phényle, R₁₅ désigne un atome d'hydrogène ou un radical alkyle inférieur tel que méthyle ou éthyle, R₁₅ désigne un atome d'hydrogène ou un radical alkyle inférieur tel que méthyle ou éthyle, R₁₇ désigne un radical alkyle inférieur tel que méthyle ou éthyle ou un radical répondant à la formule :

   -R₁₈-N(R₁₆)₂,

   R₁₈ représentant un groupement -CH₂ -CH₂-,-CH₂ -CH₂- CH₂-, -CH2-CH(CH₃)-, et R₁₆ ayant les significations mentionnées ci-dessus, ainsi que les homologues supérieurs de ces radicaux contenant jusqu'à 6 atomes de carbone.
(8) Les polymères amphotères du type -D-X-D-X-choisis parmi :
   (a) les polymères obtenus par action d'acide chloroacétique ou de chloroacétate de sodium sur les composés comportant au moins un motif de formule :

      (X) -D-X-D-X-D-

      où D désigne un radical : 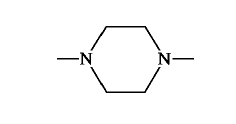 et X désigne le symbole E ou E', E ou E' identiques ou différents désignant un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote ou de soufre, 1 à 3 cycles aromatiques et/ou hétérocycliques, les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule :

      (X') -D-X'-D-X'-

      où D désigne un radical 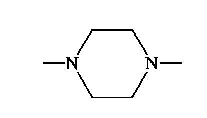 et X' désigne le symbole E ou E' et au moins une fois E', E ayant la signification indiquée ci-dessus et E' étant un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisée par réaction avec l'acide chloroacétique ou du chloroacétate de soude.
(9) Les copolymères alkyl(C₁₋₅)vinyléther/anhydride maléique modifiés partiellement par semi-amidification avec une N,N-dialkylaminoalkylamine telle que la N,N-diméthylaminopropylamine, ou par semi-estérification avec une N,N-dialcanolamine. Ces copolymères peuvent également comporter d'autres comonomères vinyliques tels que le vinylcaprolactame.

Les polymères fixants amphotères préférés selon l'invention sont ceux de la famille (3) décrite ci-dessus, tels que ceux dont la dénomination CTFA est « Octylacrylamide/acrylates/butylaminoéthyl-méthacrylate copolymer ». On peut citer à titre d'exemple les produits commercialisés sous les dénominations AMPHOMER®, AMPHOMER® LV 71 ou LOVOCRYL® 47 par la Société NATIONAL STARCH.

D'autres polymères fixants amphotères préférés sont ceux de la famille (4), comme par exemple les copolymères de méthacrylate de méthyle et de méthacrylate de diméthylcarboxyméthylammonioéthyle, commercialisés par exemple sous la dénomination DIAFORMER® Z301 par la Société SANDOZ.

Les polymères fixants anioniques ou amphotères peuvent, si nécessaire, être partiellement ou totalement neutralisés. Les agents de neutralisation sont par exemple la soude, la potasse, l'amino-2-méthylpropanol, la monoéthanolamine, la triéthanolamine ou la triisopropanolamine, les acides minéraux ou organiques tels que l'acide chlorhydrique ou l'acide citrique.

Les polymères fixants non ioniques utilisables selon la présente invention sont choisis par exemple parmi :
- les homopolymères de vinylpyrrolidone,
- les copolymères de vinylpyrrolidone et d'acétate de vinyle,
- les polyalkyloxazolines telles que les polyéthyloxazolines proposées par la Société DOW CHEMICAL sous les dénominations PEOX® 50 000 , PEOX® 200 000 et PEOX® 500 000,
- les homopolymères d'acétate de vinyle tels que le produit proposé sous le nom de APPRETAN® EM par la Société HOECHST ou le produit proposé sous le nom de RHODOPAS® A 012 par la Société RHONE POULENC ;
- les copolymères d'acétate de vinyle et d'esters acryliques tels que le produit proposé sous la dénomination RHODOPAS® AD 310 par RHONE POULENC,
- les copolymères d'acétate de vinyle et d'éthylène tels que le produit proposé sous le nom de APPRETAN® TV par la Société HOECHST,
- les copolymères d'acétate de vinyle et d'ester maléique par exemple de maléate de dibutyle tels que le produit proposé sous le nom de APPRETAN® MB EXTRA par la Société HOECHST,
- les copolymères d'éthylène et d'anhydride maléique,
- les homopolymères d'acrylates d'alkyle et les homopolymères de méthacrylates d'alkyle tels que le produit proposé sous la dénomination MICROPEARL® RQ 750 par la Société MATSUMOTO ou le produit proposé sous la dénomination LUHYDRAN® A 848 S par la Société BASF,
- les copolymères d'esters acryliques tels que par exemple les copolymères d'acrylates d'alkyle et de méthacrylates d'alkyle tels que les produits proposés par la Société ROHM & HAAS sous les dénominations PRIMAL AC-261 K et EUDRAGIT NE 30 D, par la Société BASF sous les dénominations ACRONAL® 601, LUHYDRAN® LR 8833 ou 8845, et par la Société HOECHST sous les dénominations APPRETAN® N 9213 ou N 9212,
- les copolymères d'acrylonitrile et d'un monomère non ionique choisi par exemple parmi le butadiène et les (méth) acrylates d'alkyle ; on peut citer les produits proposés sous les dénominations NIPOL® LX 531 B par la Société NIPPON ZEON ou ceux proposés sous la dénomination CJ 0610 B par la Société ROHM & HAAS,
- les polyuréthannes tels que les produits proposés sous les dénominations ACRYSOL® RM 1020 OU ACRYSOL® RM 2020 par la Société ROHM & HAAS, les produits URAFLEX® XP 401 UZ, URAFLEX® XP et 402 UZ par la Société DSM RESINS,
- les copolymères d'acrylate d'alkyle et d'uréthanne tels que le produit 8538-33 commercialisé par la Société NATIONAL STARCH,
- les polyamides tels que le produit ESTAPOR® LO 11 proposé par la Société RHONE POULENC,
- les gommes de guar non ioniques chimiquement modifiées ou non modifiées.

Les gommes de guar non ioniques non modifiées sont par exemple les produits vendus sous la dénomination VIDOGUM® GH 175 par la Société UNIPECTINE et sous la dénomination JAGUAR® C par la Société MEYHALL. Les gommes de guar non ioniques modifiées utilisables selon l'invention sont de préférence modifiées par des groupements hydroxyalkyle en C₁₋₈. On peut mentionner à titre d'exemple les groupements hydroxyméthyle, hydroxyéthyle, hydroxypropyle et hydroxybutyle.

Ces gommes de guar sont bien connues dans la technique et peuvent par exemple être préparées par réaction des oxydes d'alcènes correspondants, tels que par exemple des oxydes de propylène, avec la gomme de guar de façon à obtenir une gomme de guar modifiée par des groupements hydroxypropyle.

De telles gommes de guar non ioniques éventuellement modifiées par des groupements hydroxyalkyle sont par exemple vendues sous les dénominations commerciales JAGUAR® HP8, JAGUAR® HP60 et JAGUAR® HP120, JAGUAR® DC 293 et JAGUAR® HP 105 par la Société MEYHALL, ou sous la dénomination GALACTASOL® 4H4FD2 par la Société AQUALON.

Selon l'invention, on peut également utiliser, en tant que polymères fixants, des polymères filmogènes de type silicone greffée comprenant une partie polysiloxane et une partie constituée d'une chaîne organique non siliconée, l'une des deux parties constituant la chaîne principale du polymère et l'autre étant greffée sur ladite chaîne principale.

Ces polymères sont par exemple décrits dans les demandes de brevet EP-A-0 412 704, EP-A-0 412 707, EP-A-0 640 105 et WO 95/00578, EP-A-0582 152 et WO 93/23009 et les demandes de brevets US 4 693 935, US 4 728 571 et US 4 972 037.

Ces polymères sont de préférence anioniques ou non ioniques.

De tels polymères sont par exemple les copolymères susceptibles d'être obtenus par polymérisation radicalaire à partir du mélange de monomère formé,
a) de 50 à 90% en poids d'acrylate de tertiobutyle,
b) de 0 à 40% en poids d'acide acrylique,
c) de 5 à 40% en poids d'un macromère siliconé de formule : 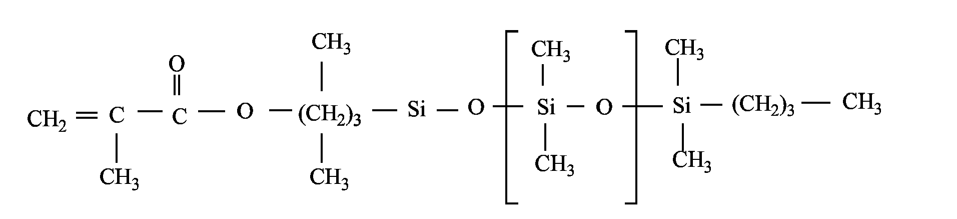 avec v étant un nombre allant de 5 à 700, les pourcentages en poids calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

On peut aussi utiliser comme polymères fixants filmogènes des polyuréthanes fonctionnalisés ou non, siliconés ou non.

Les polyuréthanes particulièrement visés par la présente invention sont ceux décrits dans les demandes de brevets EP 0 751 162, EP 0 637 600, FR 2 743 297 et EP 0 648 485 de la demanderesse, ainsi que la demande de brevet EP 0 656 021 ou WO 94/03510 de la Société BASF et EP 0 619 111 de la Société NATIONAL STARCH.

La composition de coiffage selon l'invention peut aussi comprendre au moins un composé choisi parmi les silicones sous forme soluble, dispersée, micro ou nano dispersée, volatiles ou non volatiles, les agents épaississants, les agents tensioactifs non ioniques, anioniques, cationiques et amphotères, les agents conditionneurs, les agents adoucissants, les agents anti-mousse, les agents hydratants, les agents émollients, les plastifiants, les filtres solaires hydrosolubles et liposolubles, siliconés ou non siliconés, les colorants permanents ou temporaires, les pigments minéraux ou organiques, colorés ou non colorés, les charges minérales, les argiles, les nacres ou agents, les opacifiants, les colloïdaux , les parfums, les peptisants, les conservateurs, les céramides, et pseudo-céramides, les vitamines et les provitamines dont le panthénol, les protéines, les agents séquestrants, les agents solubilisants, les agents, les agents alcanisants, les agents anticorrosion, les corps gras tels que les huiles végétales, animales, minérales et synthétiques, des agents réducteurs ou antioxydants, les agents oxydants, et leurs mélanges.

De préférence, le milieu liquide physiologiquement acceptable est constitué par de l'eau ou par un ou plusieurs solvants organiques, ou encore par un mélange d'eau et d'un ou plusieurs solvants organiques.

Le solvant organique est de préférence choisi dans le groupe comprenant les alcools de C1 à C4, les alcanes en C5 à C10, les alcools gras, les polyols modifiés ou non, les glycols, les silicones volatiles ou non, les huiles minérales organiques ou végétales, et leurs mélanges.

La composition cosmétique de l'invention est de type non rincé et peut se présenter sous diverses formes galéniques telles qu'une lotion, un spray, une mousse ou encore un gel. Le cheveu peut être chauffé avant, après ou pendant l'application de la composition contenant le matériau de haute porosité.

### EXEMPLE 1 :

1g de formule (1) est appliquée sur 2,5 g de cheveux châtains européens.

1g de formule témoin (2) est appliquée sur 2,5g de cheveux châtains européens de même nature.

L'application est réalisée en finition après séchage des cheveux.

**Formule possible de l'invention (formule 1)**

| | |
|---|---|
| Carbomer synthalen K | 0,7 % |
| Sphère poreuse à base d'acrylamide décrite dans l'exemple 1 du brevet WO 2 003 091 321 | 4 ,0 % |
| Fixate G100 | 3,0 % |
| Eau | 92,3 % |

**Formule témoin (formule 2)**

| | |
|---|---|
| Carbomer synthalen K | 0,7 % |
| Fixate G100 | 3,0 % |
| Eau | 96,3 % |

En comparant qualitativement, de manière visuelle, la fixation et la tenue de cette fixation des mèches après les avoir soumises à un mouvement oscillant horizontal pendant une demi-heure, deux heures et une demi-journée, on observe que la formule issue de l'invention (formule 1) est celle qui confère à la mèche le plus de tenue, et le plus de fixation.

## Revendications

1. Composition de coiffage comprenant, dans un milieu liquide physiologiquement acceptable, l'association :
- d'au moins un matériau polymérique hydrophile de haute porosité, à l'exclusion des polymères siliconés, et des polymères polyméthacrylate d'alkyle (PMMA), et
- d'au moins un polymère fixant.

2. Composition de coiffage selon la revendication 1, **caractérisée en ce que** le matériau polymérique hydrophile de haute porosité est solide sous forme de particules.

3. Composition de coiffage selon la revendication 1 ou 2, **caractérisée en ce que** la section des particules de matériau polymérique hydrophile est comprise entre 0,5 nm et 250 µm, et de préférence comprise entre 10 nm et 80 µm.

4. Composition de coiffage selon l'une quelconque des revendications 1 à 3, dans laquelle le matériau polymérique hydrophile possède une surface spécifique comprise entre 0,1 et 1000 m²/g.

5. Composition de coiffage selon l'une quelconque des revendications 1 à 4, **caractérisée en ce que** le matériau polymérique hydrophile de haute porosité comprend au moins un monomère hydrophile, de nature anionique, non-ionique ou cationique ou un mélange de ceux-ci.

6. Composition de coiffage selon la revendication 5, **caractérisée en ce que** le ou les monomères hydrophiles anioniques, sont choisis parmi les acides carboxyliques à insaturation éthylénique, et en particulier, l'acide acrylique, l'acide méthacrylique, l'acide itaconique, l'acide fumarique, l'acide crotonique et l'acide maléique, l'acide 2-acrylamido-2-méthylpropane-sulfonique, l'acide styrènesulfonique, l'acide vinylsulfonique et l'acide vinylphosphonique, leurs sels et leurs mélanges.

7. Composition de coiffage selon la revendication 5, **caractérisée en ce que** le ou les monomères hydrophiles non-ioniques sont choisis parmi, l'acrylamide, les acrylamides N-alkylés en C₁₋₆ ou N,N-dialkylés en C₁₋3, l'acrylate de polyéthylèneglycol, le méthacrylate de polyéthylèneglycol, le N-vinylacétamide, le N-méthyl-N-vinylacétamide, le N-vinylformamide, le N-méthyl-N-vinylformamide, les N-vinyllactames comportant un groupe cyclique de 4 à 9 atomes de carbone, l'alcool vinylique (copolymérisé sous forme d'acétate de vinyle puis hydrolysé), l'oxyde d'éthylène, l'acrylate d'hydroxyéthyle, l'acrylate d'hydroxypropyle, le méthacrylate d'hydroxyéthyle et le méthacrylate d'hydroxypropyle, le méthacrylate de méthyle, la N-acryloyl morpholine.

8. Composition de coiffage selon la revendication 5, **caractérisée en ce que** le ou les monomères hydrophiles cationiques sont choisis parmi le chlorure de diméthyldiallylammonium, le chlorure de méthylvinylimidazolium, la 2-vinylpyridine, la 4-vinylpyridique, la 2-méthyl-5-vinylpyridine, les halogénures de N-(alkyle en C₁₋₄)-4-vinylpyridinium tels que l'iodure de N-méthyl-4-vinylpyridinium, la vinylamine, les monomères de formule :
**H**_{**2**}**C=CR**_{**1**}**-CO-X**_{**2**}
dans laquelle:
R₁ représente un atome d'hydrogène ou un groupe méthyle,
X₂ représente un groupe hydrocarboné linéaire ou ramifié en C₁₋₆ portant au moins une fonction amine primaire, secondaire ou tertiaire ou au moins un atome d'azote quaternaire, ou un groupe de formule NHR₂ ou de formule NR₂R₃ où R₂ et R₃ représentent indépendamment l'un de l'autre chacun un groupe hydrocarboné en C₁₋₆, linéaire ou ramifié, portant au moins une fonction amine primaire, secondaire ou tertiaire ou au moins un atome d'azote quaternaire.

9. Composition de coiffage selon l'une quelconque des revendications 1 à 8, **caractérisée en ce que** le matériau polymérique hydrophile de haute porosité contient de 0,1 à 40% en poids d'un ou plusieurs monomères hydrophobes choisis parmi, des monomères vinylaromatiques, des diènes, des dérivés alkylés des diènes, le chloroprène, des acrylates d'alkyle en C₁₋₁₀, d'aryle en C₆₋₁₀ ou d'aralkyle en C₇₋₂₀ et des méthacrylates d'alkyle en C₁₋₁₀, d'aryle en C₆₋₁₀ ou d'aralkyle en C₇₋₂₀, l'acétate de vinyle, les vinyléthers de formule CH₂=CH-O-R et les allyléthers de formule CH₂=CH-CH₂-O-R où R représente un groupe alkyle en C₁₋₆, l'acrylonitrile, le chlorure de vinyle, le chlorure de vinylidène, la caprolactone, l'éthylène, le propylène, des monomères vinyliques fluorés ou à chaîne perfluorée, et des mélanges de ceux-ci.

10. Composition de coiffage selon l'une quelconque des revendications 1 à 9, **caractérisée en ce que** le polymère fixant est choisi parmi les polymères fixants anioniques, cationiques, amphotères ou non ioniques.

11. Composition de coiffage selon l'une quelconque des revendications 1 à 9, **caractérisée en ce qu'**elle comprend en outre un composé choisi parmi les silicones sous forme soluble, dispersée, micro ou nano dispersée, volatiles ou non volatiles, les agents épaississants, les agents tensioactifs non ioniques, anioniques, cationiques et amphotères, les agents conditionneurs, les agents adoucissants, les agents anti-mousse, les agents hydratants, les agents émollients, les plastifiants, les filtres solaires hydrosolubles et liposolubles, siliconés ou non siliconés, les colorants permanents ou temporaires, les pigments minéraux ou organiques, colorés ou non colorés, les charges minérales, les argiles, les nacres ou agents, les opacifiants, les colloïdaux , les parfums, les peptisants, les conservateurs, les céramides, et pseudo-céramides, les vitamines et les provitamines dont le panthénol, les protéines, les agents séquestrants, les agents solubilisants, les agents, les agents alcanisants, les agents anticorrosion, les corps gras tels que les huiles végétales, animales, minérales et synthétiques, des agents réducteurs ou antioxydants, les agents oxydants, et leurs mélanges.

12. Composition de coiffage selon l'une quelconque des revendications 1 à 9, **caractérisée en ce que** le milieu liquide physiologiquement acceptable est constitué par de l'eau ou par un ou plusieurs solvants organiques, ou encore par un mélange d'eau et d'un ou plusieurs solvants organiques.

13. Composition de coiffage selon la revendications 12, **caractérisée en ce que** le solvant organique est choisi dans le groupe comprenant les alcools en C1 à C4, les alcanes en C5 à C10, les alcools gras, les polyols modifiés ou non, les glycols, les silicones volatiles ou non, les huiles minérales organiques ou végétales, et leurs mélanges.

14. Utilisation d'un polymère hydrophile de haute porosité pour améliorer la fixation de la coiffure.
